# EUROPÄISCHE PATENTSCHRIFT

(11) **EP 3 054 850 B1**
(45) Veröffentlichungstag und Bekanntmachung des Hinweises auf die Patenterteilung: **28.06.2017**
(21) Anmeldenummer: 14806162.5
(22) Anmeldetag: 09.10.2014
(51) Int. Cl.: A61B 5/15, B01L 3/14, B01L 3/00, G01N 33/49

(54) **ABNAHMEBAUGRUPPE, INSBESONDERE FÜR BLUTPROBEN**
COLLECTION ASSEMBLY, IN PARTICULAR FOR BLOOD SAMPLES
MODULE DE PRÉLÈVEMENT, EN PARTICULIER D'ÉCHANTILLONS SANGUINS

(30) Priorität: 11.10.2013 AT 506582013
(43) Veröffentlichungstag der Anmeldung: 17.08.2016
(73) Patentinhaber: Greiner Bio-One GmbH, 4550 Kremsmünster (AT)
(72) Erfinder: BAUER, Christian, A-4550 Kremsmünster (AT); KOFLER, Georg, A-4565 Inzersdorf (AT)
(74) Vertreter: Burger, Hannes
(86) Internationale Anmeldenummer: PCT/AT2014/050237
(87) Internationale Veröffentlichungsnummer: WO 2015/051389

(56) Entgegenhaltungen:
- EP-B1- 1 066 882
- US-A- 4 092 113
- US-A- 5 288 466
- US-A- 5 384 096
- US-A1- 2012 141 341

## Beschreibung

Die Erfindung betrifft eine Abnahmebaugruppe, insbesondere für Blutproben, wie diese im Anspruch 1 beschrieben ist.

Die US 4,092,113 A beschreibt eine Abnahmebaugruppe für Blutproben mit einem Probenbehälter, einer diesen verschließenden Verschlussvorrichtung sowie ein den Probenbehälter aufnehmendes Trägerröhrchen. Der Probenbehälter weist ein offenes Ende, ein durch einen Boden verschlossenes Ende sowie eine sich zwischen den beiden Enden erstreckende Seitenwand mit einer Außenfläche und einer Innenfläche auf. Die Seitenwand sowie der Boden definieren einen Sammelraum für die Blutprobe. Der Probenbehälter weist weiters an seinem mit einer Stirnfläche begrenzten offenen Ende einen die Seitenwand radial überragenden Flansch sowie eine Ringschürze auf. Die Ringschürze ist an einem von der Seitenwand abgewendeten Flanschende des Flansches angeordnet und erstreckt sich in axialer Richtung ausgehend vom Flanschende auf das verschlossene Ende. Der Probenbehälter, der Flansch und die Ringschürze bilden gemeinsam ein einstückiges Bauteil aus. Zwischen der Außenfläche der Seitenwand und einer der Seitenwand zugewendeten inneren Ringschürzenfläche der Ringschürze ist ein in etwa rohrformiger Freiraum zur Aufnahme des Trägerröhrchens ausgebildet. Ein Kappenmantel der Verschlusskappe übergreift außenseitig die Ringschürze des Probenbehälters. Ein rohrförmig ausgebildeter Ansatz ragt in Axialrichtung vom Flansch auf die vom verschlossenen Ende abgewendete Richtung vor und ist mit einem Außengewinde versehen.

An der Innenfläche des Kappenmantels ist ein Innengewinde angeordnet. In der Verschlusskappe ist im Bereich von deren Stirnwand ein durchstechbares, scheibenförmig ausgebildetes Dichtelement angeordnet, an welchem der vom Flansch aufragend ausgebildete rohrförmige Ansatz in der Verschlussstellung anliegt.

Eine ähnlich ausgebildete Abnahmebaugruppe für Blutproben ist aus der US 2012/0141341 A1 bekannt geworden. Die Abnahmebaugruppe umfasst einen Probenbehälter, welcher in ein Trägerröhrchen eingesetzt ist und mittels einer Verschlussvorrichtung verschlossen ist. Der Probenbehälter weist einen flanschförmig ausgebildeten Ansatz auf, welcher an einer Stirnwand des offenen Endes des Trägerröhrchens anliegt. Weiters ist im Bereich der offenen Enden zwischen dem Probenbehälter und dem Trägerröhrchen eine Rastvorrichtung angeordnet, um eine gegenseitige Lagenfixierung und Halterung des Probenbehälters im Trägerröhrchen zu erzielen. Der Kappenmantel der Verschlussvorrichtung übergreift das offene Ende des Trägerröhrchens und ist im Bereich von dessen Außenwand daran verrastet gehalten.

Aus der EP 1 066 882 B1 ist eine gattungsgemäße Anordnung zur Probensammlung für Blutproben, mit einem Probenbehälter, der ein offenes Ende, ein durch einen Boden verschlossenes Ende und eine sich zwischen dem offenen Ende und dem verschlossenen Ende erstreckende Seitenwand mit einer Außenfläche und einer Innenfläche aufweist. Die Seitenwand ist im Bereich des offenen Endes mit einer Stirnfläche begrenzt, wobei, die Seitenwand sowie der Boden einen Sammelraum definieren, bekannt geworden. Der Probenbehälter ist mit einer Verschlussvorrichtung verschließbar und weist eine Verschlusskappe, sowie ein an der Verschlusskappe gehaltenes, durchstechbares Septum auf. Die Verschlussvorrichtung schließt in deren Verschlussstellung den Sammelraum des Probenbehälters im Bereich seines offenen Endes ab. Der äußere Kappenmantel des Verschlusskappe übergreift außenseitig das offene Ende des Probenbehälters und stützt sich an einem vom offenen Ende distanzierten Stützring ab. der innere Kappenmantel ragt in den Sammelraum hinein und liegt mit einem radial vorragenden Ringansatz an der Innenfläche der Seitenwand des Probenbehälters an. Am dem dem Boden zugewendeten offenen Stirnende des inneren Kappenmantels ist ein durchstechbares Septum angeordnet.

Weitere derart ausgebildete Anordnungen zur Probensammlung für Blutproben sind auch aus der US 5,288,466 A sowie der US 5,384,096 A bekannt geworden.

Nachteilig ist bei all diesen Probensammlungsanordnungen, dass diese nicht in allen Anwendungsfällen einfach handhabbar waren und eine Zentrifugation der aufgenommenen Körperflüssigkeit nur unter Zuhilfenahme von zusätzlichen Bauteilen möglich war. Dies deshalb, da derartige Probensammlungsanordnungen bezüglich standardisierten-Blutprobenabnahmeröhrchen dazu geringere Abmessungen aufweisen.

Der vorliegenden Erfindung liegt die Aufgabe zugrunde, eine Abnahmebaugruppe insbesondere für die Abnahme von Kapillarblut zu schaffen, welche universellere Einsatzmöglichkeiten für den Anwender bietet.

Diese Aufgabe der Erfindung wird durch die Merkmale des Anspruches 1 gelöst.

Der sich durch die Merkmale des Anspruches 1 ergebende Vorteil liegt darin, dass dadurch eine doppelwandige Kappe geschaffen wird, bei welcher der zwischen dem äußeren Kappenmantel und dem inneren Kappenmantel gebildete rohr- bzw. ringförmige Freiraum zur Aufnahme des zumeist lippenförmig ausgebildeten Aufnahmeansatzes des Probenbehälters dient. Des Weiteren wird durch den inneren Kappenmantel eine definierte Zugangsöffnung ausgehend von der Verschlusskappe hin zum Sammelraum geschaffen. Weiters kann der äußere Kappenmantel derart ausgebildet werden, dass dieser zur leichteren Bedienung und Handhabung der Verschlussvorrichtung mit entsprechenden Hilfselementen versehen werden kann. Weiters kann so im Übergangsbereich zwischen dem Flansch sowie der Ringschürze eine ausreichende Abstützung der Verschlussvorrichtung am Probenbehälter gewährleistet werden. Damit kann aber auch im Zusammenwirken mit der Ringschürze in etwa die äußere Raumform von standardisierten Verschlusskappen, wie diese bei Blutprobenröhrchen eingesetzt werden, ausgebildet werden.

Darüber hinaus kann so ein unbeabsichtigtes Lösen der Verschlussvorrichtung vom Probenbehälter bis hin zur Überschreitung einer vordefinierten Lösekraft verhindert werden. Damit kann aber auch bei sich in der Verschlussstellung befindlicher Verschlussvorrichtung ein unbeabsichtigtes Lösen der Verschlussvorrichtung, wie beispielsweise leichte Stöße, die beim Umfallen der befüllten Abnahmebaugruppe entstehen, verhindert werden. Durch die in etwa gleiche Ausbildung der Außenabmessungen kann dadurch eine zusammengehörige Baueinheit geschaffen werden, bei welcher bei aufgesetzter Verschlussvorrichtung im Zusammenwirken mit der Ringschürze eine maßlich durchgehende Baueinheit geschaffen wird. Wenn zusätzlich noch im Bereich der offenen Stirnseite der Ringschürze, welche dem Boden zugewendet ist, an dessen Außenseite über den Umfang zumindest abschnittsweise ein in radialer Richtung über die Ringschürze vorragender Bund angeordnet ist, kann so eine direkte Auflage des äußeren Kappenmantels der Verschlusskappe an einer Auflagefläche in liegender Stellung derselben vermieden werden. Somit kann auch bei einer liegenden Stellung der Abnahmebaugruppe ein unbeabsichtigtes Lösen der Verschlussvorrichtung vom Probenbehälter verhindert werden.

Durch das distanzierte Anordnen des Septums von der Stirnwand ist dadurch bei aufgesetzter Verschlussvorrichtung der Zugang zum Sammelraum unterbrochen. Darüber hinaus wird so eine Einheit geschaffen, welche gemeinsam vom Probenbehälter abgenommen und wiederum zu dessen Verschluss aufgesetzt werden kann.

Durch das Vorsehen einer zusätzlichen Ringschürze, welche über einen die Seitenwand des Probenbehälters radial überragenden Flansch mit diesem verbunden ist, wird direkt am Probenbehälter ein kappenähnlicher Bauteil vorgesehen, mittels welchem die mehrfache, universellere Einsatzmöglichkeit geschaffen wird. So kann bei einer ersten Anwendung nach erfolgter Aufnahme der Körperflüssigkeit, insbesondere des Kapillarblutes, die Abnahmebaugruppe ohne Zuhilfenahme zusätzlicher Bauteile in eine standardisierte Zentrifugationsvorrichtung eingesetzt werden. Durch das Vorsehen des Flansches in Verbindung mit der Ringschürze kann so eine einwandfreie Kraftübertragung und damit verbundene Abstützung der Abnahmebaugruppe direkt an der jeweiligen Zentrifuge erfolgen. Darüber hinaus wird aber auch damit die Möglichkeit geschaffen, eine ausreichende Abstützung sowie gegebenenfalls Halterung der Verschlussvorrichtung am Probenbehälter zu gewährleisten. Bei aufgesetzter Verschlussvorrichtung auf den Probenbehälter kann so im Zusammenwirken mit der Ringschürze eine kappenähnliche Form geschaffen werden, wie diese bei in der Medizintechnik standardisierten Kappenabmessungen bei Blutprobenabnahmeröhrchen üblich sind.

Eine weitere Anwendungsmöglichkeit ergibt sich noch dadurch, den zwischen der inneren Ringschürzenfläche der Ringschürze und der Außenfläche der Seitenwand ausgebildeten, rohrförmigen Freiraum dazu zu verwenden, den Probenbehälter in ein standardisiertes Blutprobenentnahmeröhrchen bzw. Trägerröhrchen einsetzen zu können, um so eine Halterung des Probenbehälters an diesem Röhrchen zu ermöglichen. Damit wird so die universellere Einsatzmöglichkeit des Probenbehälters geschaffen, welcher entweder für sich alleine oder in Verbindung mit einem zusätzlichen Trägerröhrchen einer Zentrifugation zugeführt werden kann. Des Weiteren wird aber auch ein einfacherer und kostengünstigerer Herstellungsvorgang ermöglicht, da keine zusätzlichen Teile hergestellt werden müssen, um eine funktionsfähige Abnahmebaugruppe zu schaffen.

Vorteilhaft ist auch eine weitere Ausfiihrungsform nach Anspruch 2, da dadurch die Möglichkeit geschaffen wird, den Probenbehälter über die daran angeordnete Ringschürze mit einer weiteren an einem Trägerröhrchen angeordneten Gewindeanordnung verschrauben zu können. Dadurch kann gegenüber einer Steck- bzw. Klemmverbindung eine noch sicherere gegenseitige Verbindung geschaffen werden.

Vorteilhaft ist weiters eine Ausbildung nach Anspruch 3, da dadurch der Einfüllvorgang ohne zusätzliche Bauteile in den dafür vorgesehenen Sammel- bzw. Aufnahmeraum des Probenbehälters ermöglicht wird. Damit ist für den Einfüllvorgang lediglich die Verschlussvorrichtung abzunehmen und der Einfüllvorgang kann unmittelbar anschließend daran erfolgen. Durch den Wegfall von zusätzlichen Bauteilen können so die Herstell- sowie Materialkosten der Abnahmebaugruppe reduziert werden.

Durch die Ausbildung nach Anspruch 4 ist es möglich, einen einwandfreien Einfüllvorgang durchführen zu können. So kann nicht nur während des Einfüllvorganges ein ausreichender Blickkontakt zu der Abnahmestelle gewährleistet werden, sondern es ist auch ein ungehindertes Hineinfließen der abzunehmenden Körperflüssigkeit, insbesondere des Kapillarblutes, in den Sammelraum möglich.

Nach einer anderen Ausführungsvariante gemäß Anspruch 5 wird die Möglichkeit geschaffen, dass ein Abstellen des Probenbehälters direkt auf dem rahrförmigen Ansatz auf einer definierten Unterlage bzw. Abstellfläche ermöglicht wird. Dies erfolgt dann, wenn der rohrförmige Ansatz auf seiner vom Boden abgewendeten Seite in einer senkrecht bezüglich der Längsachse ausgerichteten Ebene endet.

Durch die Weiterbildung nach Anspruch 6 wird erreicht, dass das Septum an seiner äußeren Umfangsseite mit der Innenfläche der Seitenwand in der Verschlussstellung einen durchgehenden, vollkommenen Abschluss des Sammelraums ausbildet. Damit kann bis zur Abnahme der Verschlussvorrichtung ein Eindringen von Verschmutzungen gesichert verhindert werden sowie auch die Sterilität bis zur ersten Öffnung gewährleistet werden.

Vorteilhaft ist auch eine Ausbildung nach Anspruch 7, da dadurch eine noch exaktere gegenseitige Ausrichtung der Verschlussvorrichtung, insbesondere der Verschlusskappe, bezüglich des Probenbehälters und der daran angeordneten Ringschürze erfolgen kann.

Nach einer vorteilhaften Weiterbildung gemäß Anspruch 8 wird so die Möglichkeit geschaffen, die Abnahmebaugruppe auch einem automatisierten Analysevorgang mit standardisierten Analysegeräten zuführen und dort die Untersuchungen durchführen zu können. Dies deshalb, da bei derartigen automatisierten Analyseverfahren standardisierte Abmessungen in Bezug auf Länge, Querschnittsform sowie Durchmesser bzw. Außenabmessungen erforderlich sind. Durch das Einsetzen des Probenbehälters in das Trägerröhrchen, kann so eine Abnahmebaugruppe bereitgestellt werden, welche den standardisierten Abmessungen von Blutprobenentnahmeröhrchen entspricht. Des Weiteren kann so aber auch der Probenbehälter, insbesondere dessen Seitenwand, mit einer Abmessung ausgebildet werden, welche dazu geeignet ist, in das Innere des Trägerröhrchen eingesetzt bzw. darin aufgenommen zu werden. So kann aber auch bei gleichbleibendem Füllvolumen gegenüber bisher eingesetzten Probenbehältern aufgrund der Vergrößerung der Außenabmessung eine dazu kürzere Distanzierung des Bodens vom offenen Ende erreicht werden, als dies bei bisher üblichen Abnahmevorrichtungen der Fall war.

Vorteilhaft ist auch eine Ausbildung nach Anspruch 9, da damit eine zusammengehörige und im Gebrauch auch eine stabile Verbindung aufweisende Abnahmebaugruppe geschaffen werden kann. Damit wird es möglich, den Probenbehälter ohne zusätzliche Bauteile mit einem standardisierten Blutprobenröhrchen zu verbinden und so die zuvor beschriebenen automatisierten Analysen bzw. Untersuchungen des Probeninhalt durchführen zu können.

Schließlich ist aber auch eine Ausbildung, wie in den Ansprüchen 10 und 11 beschrieben, möglich, da so eine Trennung erschwert bzw. überhaupt unterbunden werden kann. Dies dient dazu, dass eine Verwechslung von Proben möglich ist, wenn z.B. nur am Trägerröhrchen ein Informationsträger angeordnet bzw. aufgebracht ist. Damit kann die im Probenbehälter aufgenommene Probe stets eindeutig einem Patienten zugeordnet werden. So kann auf eine Kennzeichnung
direkt am Probenbehälter verzichtet werden. Da die Abmessungen des Probenbehälters relativ bezüglich des Trägerröhrchens kleiner ausgebildet sind, steht für das Anbringen des Informationsträgers am Trägerröhrchen ein größeres Platzangebot zur Verfügung. Damit können standardisierte Informationsträger Anwendung finden.

Zum besseren Verständnis der Erfindung wird diese anhand der nachfolgenden Figuren näher erläutert.

Es zeigen jeweils in stark vereinfachter, schematischer Darstellung:
- Fig. 1: einen mit einer Verschlussvorrichtung verschlossenen Probenbehälter, in schaubildlicher Darstellung;
- Fig. 2: den Probenbehälter mit teilweise abgenommener Verschlussvornchtung nach Fig. 1, im Axialschnitt;
- Fig. 3: den Probenbehälter mit Verschlussvorrichtung nach den Fig. 1 und 2, der in ein zusätzliches Trägerröhrchen eingesetzt ist, im Axialschnitt;
- Fig. 4: die Abnahmebaugruppe nach den Fig. 1 bis 3, im Axialschnitt sowie voneinander getrennter Stellung der Einzelbauteile;
- Fig. 5: den in ein Trägerröhrchen eingesetzten Probenbehälter nach den Fig. 1 bis 4, mit einer zusätzlichen Verdrehsicherungsvorrichtung, in Draufsicht geschnitten.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

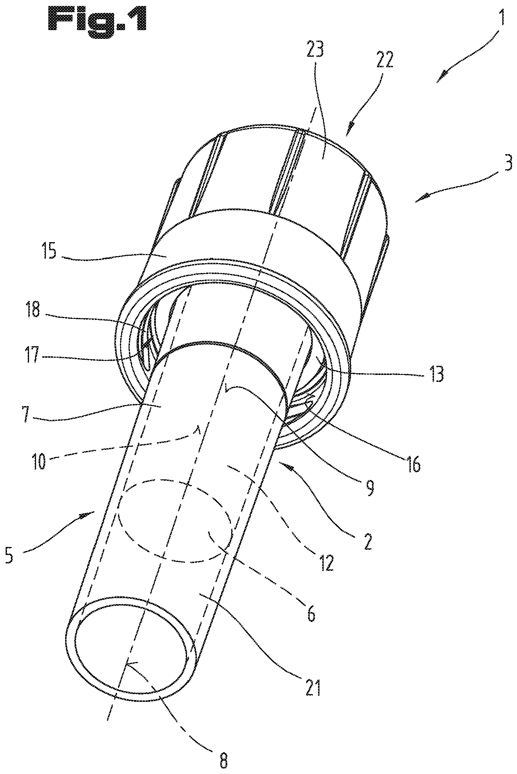

In den Fig. 1 bis 4 ist eine Abnahmebaugruppe 1 in unterschiedlichen Konfigurationen dargestellt, welche insbesondere zur Abnahme von Körperflüssigkeiten, wie Blutproben, dient. Diese hier gezeigte Abnahmebaugruppe 1 weist gegenüber üblich verwendeten Blutprobenentnahmeröhrchen in den zumeist normierten Standardabmessungen eine dazu geringere Außenabmessung sowie ein dazu geringeres Aufnahmevolumen auf. Diese Abnahmebaugruppe 1 wird bevorzugt für die Abnahme von Kapillarblut verwendet, welches in nur geringeren Abnahmemengen abgenommen und für nachfolgende Untersuchungen in der Abnahmebaugruppe 1 bevorratet wird.

Die hier dargestellte Abnahmebaugruppe 1 umfasst einen Probenbehälter 2 sowie eine Verschlussvorrichtung 3.

Der Probenbehälter 2 weist seinerseits ein offenes Ende 4 sowie ein verschlossenes Ende 5 auf. Das verschlossene Ende 5 kann dabei durch einen Boden 6 verschlossen ausgebildet sein. Zwischen dem offenen Ende 4 und dem verschlossenen Ende 5 erstreckt sich eine Seitenwand 7. Weiters erstreckt sich zwischen dem offenen Ende 4 und dem verschlossenen Ende 5 eine Längsachse 8, welche bevorzugt auch als Mittellängsachse bezeichnet werden kann. Die Querschnittsform des Probenbehälters 2 kann unterschiedlichst gewählt sein, wobei jedoch zumeist als bevorzugte Querschnittsform ein kreisrunder Querschnitt gewählt wird. Um eine Entformung des bevorzugt in einem Spritzgussvorgang hergestellten Probenbehälters 2 zu ermöglichen, sind entsprechende Formschrägen vorzusehen, wie dies bei derartigen Bauteilen üblich ist. Bevorzugt wird weiters als Werkstoff für den Probenbehälter ein Kunststoffmaterial gewählt. Dabei kann der Werkstoff überwiegend durchsichtig bis glasklar ausgebildet sein und aus der Gruppe von PP (Polypropylen), PS (Polystyrol), PET (Polyethylenterephthalat), PE (Polyäthylen), PA (Polyamid), PC (Polycarbonat) gewählt sein.

Die Seitenwand 7 des Probenbehälters 2 weist weiters eine Außenfläche 9 sowie eine in einer Wandstärke der Seitenwand 7 davon distanzierte Innenfläche 10 auf. Die Seitenwand 7 ist weiters im Bereich des offenen Endes 4 mit einer Stirnfläche 11 begrenzt. Die Seitenwand 7 mit ihrer Innenfläche 10 definiert gemeinsam mit dem Boden 6 einen Sammelraum 12, welcher zur Aufnahme der abzunehmenden bzw. einzufüllenden Körperflüssigkeit, insbesondere Blut, besonders bevorzugt Kapillarblut, dient. Der Probenbehälter 2 umfasst weiters an seinem mit der Stirnfläche 11 begrenzenden offenen Ende 4 einen die Seitenwand 7 in radialer Richtung bezüglich der Längsachse 8 überragenden Flansch 13. Der Flansch 13 weist an seinem von der Seitenwand 7 bzw. von der Längsachse 8 abgewendeten Seite ein äußeres Flanschende 14 auf.

An diesem von der Seitenwand 7 abgewendeten Flanschende 14 ist weiters eine Ringschürze 15 angeordnet bzw. ausgebildet, wobei sich die Ringschürze 15 ausgehend vom Flanschende 14 in axialer Richtung auf den Boden bzw. das verschlossene Ende 5 des Probenbehälters 2 hin erstreckt. Die Ringschürze 15 weist eine der Seitenwand 7 bzw. deren Außenfläche 9 zugewendete, innere Ringschürzenfläche 16 auf. Weiters ist zwischen der Außenfläche 9 und der Seitenwand 7 und der der Seitenwand 7 zugewendeten, inneren Ringschürzenfläche 16 der Ringschürze 15 ein in etwa rohrförmig ausgebildeter Freiraum 17 ausgebildet. Dieser über den Umfang durchlaufende Freiraum 17 erstreckt sich in Axialrichtung ausgehend von dem vom Flansch 13 abgewendeten Ende der Ringschürze 15 bis hin zum Flansch 13. Damit ist in Axialrichtung der Freiraum 17 im Bereich des offenen Endes 4 durch den Flansch 13 abgeschlossen bzw. begrenzt.

Der Probenbehälters 2, der Flansch 13 sowie die Ringschürze 15 bilden bevorzugt ein einstückiges Bauteil aus, welcher zumeist in einem Spritzgussvorgang in einem Spritzgusswerkzeug in bekannter Art und Weise hergestellt wird.

Die Funktion des Flansches 13 mit der von der Außenfläche 9 der Seitenwand 7 distanziert angeordneten Ringschürze 15 wird in einer der nachfolgenden Figuren noch näher erläutert werden.

Bei einer möglichen Weiterbildung ist vorgesehen, dass an der inneren Ringschürzenfläche 16 der Ringschürze 15 eine erste Gewindeanordnung 18 angeordnet oder ausgebildet ist. Dabei sei erwähnt, dass unter Gewindeanordnung ein ein- oder mehrgängiges Gewinde sowie auch mehrere Gewindegänge bzw. Gewindesegmente verstanden werden können. So wäre es beispielsweise möglich, die erste Gewindeanordnung 18 derart auszubilden, wie diese in der EP 1 711 412 B1 der gleichen Anmelderin für die dort beschriebene Kappe zum Verschließen des Aufnahmebehälters beschrieben worden ist

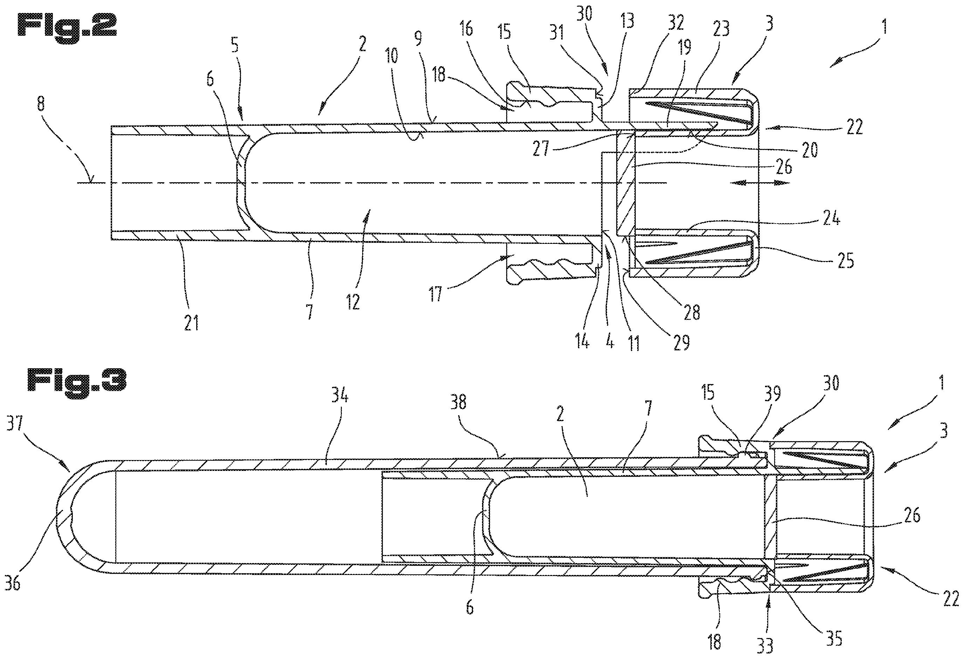

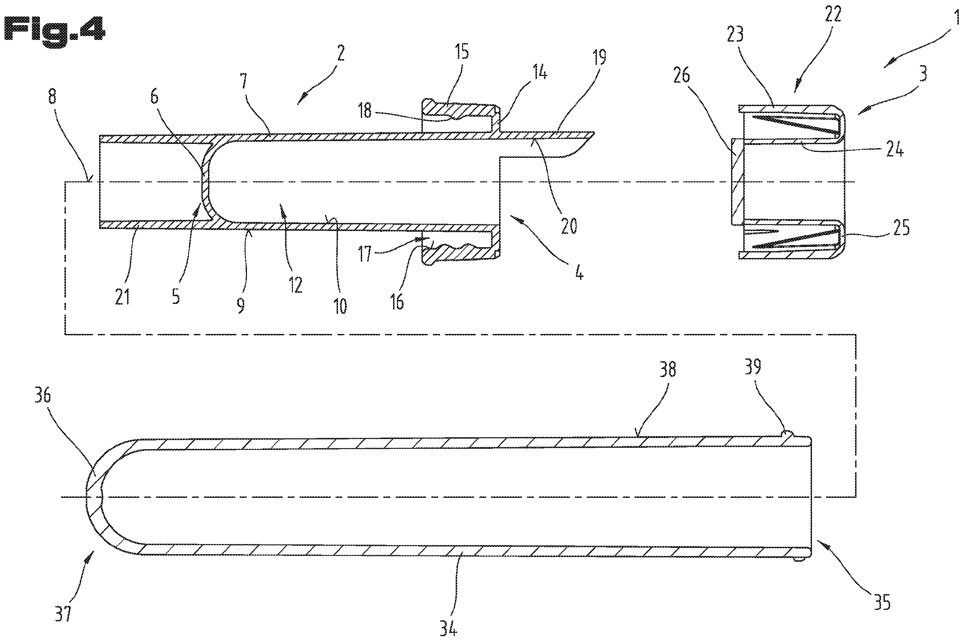

So wäre es möglich, auch das in der EP 1 711 412 B1 beschriebene Verfahren zum Zusammenbau der hier beschriebenen Abnahmebaugruppe 1 anzuwenden. Die näheren Erläuterungen dazu folgen bei der Beschreibung zu den Fig. 3 und 4.

Um ein Einfüllen der Probe, insbesondere des Blutes, in den Sammelraum 12 des Probenbehälters 2 zu erleichtern, kann ein insbesondere lippenförmig ausgebildeter Aufnahmeansatz 19 vorgesehen sein. Der Aufnahmeansatz 19 ist bevorzugt an der Stirnfläche 11 angeordnet und mit der Seitenwand 7 einstückig verbunden. Dieser überragt auch noch die Stirnfläche 11 des offenen Endes 4 in Axialrichtung auf die vom Boden 6 abgewendete Richtung. Dabei ist es möglich, dass sich der Aufnahmeansatz 19 nur über einen Teilabschnitt des Umfangs der Seitenwand 7 erstreckt. Eine dem Sammelraum 12 zugewendete Aufnahmeansatzfläche 20 geht in Axialrichtung gesehen in etwa geradlinig, insbesondere ebenflächig, in die durch die Seitenwand 7 gebildete Innenfläche 10 über. Damit werden ein ebenflächiger Übergang und ein vereinfachtes Einfüllen ohne zusätzliche störende Rückhaltekante ermöglicht.

Weiters kann der Probenbehälter 2 an seinem verschlossenen Ende 5 einen rohrförmig ausgebildeten Ansatz 21 aufweisen, welcher das mit dem Boden 6 verschlossene Ende 5 in Axialrichtung auf die vom offenen Ende 4 abgewendete Richtung überragt. Zumeist endet der Ansatz 21 an seiner vom offenen Ende 4 abgewendeten Seite in einer senkrecht zur Längsachse 8 ausgerichteten Ebene. Diese Ebene bildet beispielsweise eine Standfläche für die Abnahmebaugruppe 1 aus, um diese auf einer nicht näher dargestellten Abstellfläche, wie beispielsweise einem Labortisch oder ähnlichem, in senkrechter Ausrichtung abstellen zu können. Durch die ebenflächige Ausbildung des Ansatzes 21 wird auch eine gewisse Distanzierung des Bodens 6 vom ebenfalls offen ausgebildeten Ansatzende erzielt, wodurch in Abhängigkeit vom Überstand des Ansatzes 21 in axialer Richtung über den Boden 6 hinaus, die Gesamtlänge des Probenbehälters 2 festgelegt werden kann. Damit wird es aber auch möglich, beispielsweise bei gleicher Bauteillänge des Probenbehälters 2 den Boden 6 in einem vorgegebenen Abstand ausgehend von der Stirnfläche 11 im Inneren der Seitenwand 7 anzuordnen, um so das Aufnahmevolumen auf den jeweils gewünschten Einsatzfall exakt anpassen bzw. abstimmen zu können.

Die Verschlussvorrichtung 3 umfasst ihrerseits eine Verschlusskappe 22 mit einem äußeren Kappenmantel 23, einen in radialer Richtung bezüglich der Längsachse 8 distanziert angeordneten, inneren Kappenmantel 24 sowie eine die beiden Kappenmäntel 23, 24 in radialer Richtung verbindende Stirnwand 25. Bevorzugt sind die beiden Kappenmäntel 23, 24 zueinander konzentrisch bezüglich der Längsachse 8 ausgerichtet. Die Verbindung der beiden Kappenmäntel 23, 24 in radialer Richtung durch die Stirnwand 25 erfolgt bei aufgesetzter Verschlusskappe 22 auf das offene Ende 4 des Probenbehälters 2 auf der vom offenen Ende 4 abgewendeten Seite der Verschlusskappe 22. Der innere Kappenmantel 24 bildet eine Durchgangsöffnung aus, um so auch bei aufgesetzter Verschlussvorrichtung 3 einen Zugang in den Sammelraum 12 zu ermöglichen.

Die Verschlussvorrichtung 3 umfasst bei diesem Ausführungsbeispiel zusätzlich zur Verschlusskappe 22 auch noch ein an der Verschlusskappe 22 gehaltenes, durchstechbares Septum 26. Das Septum 26 dient dazu, bei aufgesetzter Verschlussvorrichtung 3 den Sammelraum 12 gegenüber der äußeren Umgebung abzudichten. So kann das durchstechbares Septum 26 auch aus einem selbsttätig wiederverschließenden Werkstoff gebildet sein. Als Werkstoff kann einer aus der Gruppe von Brombutylkautschuk, TPE, Kautschuk, Gummi gewählt werden. Als bevorzugter Werkstoff kann TPE eingesetzt werden, da dieser auch in einem Spritzgussvorgang verarbeitet werden kann. Das Septum 26 ist dabei so ausgebildet, dass dieses in der Verschlussstellung der Verschlussvorrichtung 3 einen flüssigkeits- und/oder gasdichten Verschluss des Sammelraums 12 im Bereich des offenen Endes 4 ausbildet.

Bei diesem Ausführungsbeispiel ist das Septum 26 an einem von der Stirnwand 25 distanziert angeordneten und offen ausgebildeten Stirnende 27 des inneren Kappenmantels 24 angeordnet oder ausgebildet. Weiters weist das Septum 26 eine radial umlaufende Dichtfläche 28 auf. In der Verschlussstellung wird das Septum 26 in das offene Ende 4 des Probenbehälters 2 eingesetzt, sodass die Dichtfläche 28 in der Verschlussstellung an der Innenfläche 10 der Seitenwand 7 anliegt. Dieses Anliegen erfolgt in insbesondere abdichtender Weise. Damit kann der Sammelraum 12 bzw. Aufnahmeraum auch im Bereich des offenen Endes 4 bei aufgesetzter Verschlussvorrichtung 3 abgeschlossen werden. Weiters erstreckt sich das Septum 26 vollflächig über das gesamte offene Ende 4 des Probenbehälters 2 im Bereich seiner Innenfläche 10. Das Septum 26 ist im vorliegenden Ausführungsbeispiel als scheibenförmiger Bauteil ausgebildet und ist mit dem inneren Kappenmantel 24 verbunden bzw. an diesem angeordnet. Bei unterschiedlicher Werkstoffwahl zwischen der Verschlusskappe 22 und dem Septum 26 kann die Verbindung durch Verkleben, Verschweißen oder andere thermische oder chemische Verbindungsvorgänge erfolgen. Unabhängig davon wäre es aber auch möglich, bei Wahl unterschiedlicher Werkstoffe die Verschlussvorrichtung 3 in einem Co-Spritzgussvorgang herzustellen. Eine weitere Möglichkeit bestünde aber auch noch darin, die gesamte Verschlussvorrichtung 3 umfassend die Verschlusskappe 22 sowie das Septum 26 aus ein und demselben Werkstoff auszubilden.

Der äußere Kappenmantel 23 weist an seiner der Stirnwand 25 gegenüberliegenden Seite ebenfalls ein offenes Stirnende 29 auf. Bei sich in der Verschlussstellung befindlicher Verschlussvornchtung 3 liegt das offene Stirnende 29 des äußeren Kappenmantels 23 am Flansch 13 oder der Ringschürze 15 an. Dies erfolgt zumeist im Eck- bzw. Übergangsbereich zwischen den zuvor beschriebenen Bauteilen.

Wie nun besser aus einer Zusammenschau der Fig. 2 und 3 zu ersehen ist, kann zwischen dem offenen Stirnende 29 des äußeren Kappenmantels 23 und dem Flansch 13 oder der Ringschürze 15 eine Zentrieranordnung 30 vorgesehen sein. Die Zentrieranordnung 30 umfasst zusammenwirkende, erste und zweite Zentrierelemente 31, 32, welche in der Verschlussstellung entsprechend zusammenwirken. So ist bei diesem Ausführungsbeispiel das erste Zentrierelement 31 eine als nach außen offene Zentriernut ausgebildet, welche im Flansch 13 oder der Ringschürze 15 in deren Übergangsbereich angeordnet bzw. ausgebildet ist. Bevorzugt ist das erste Zentrierelement 31 durchlaufend über den gesamten Umfang des Flansches 13 bzw. der Ringschürze 15 ausgebildet.

Das zweite Zentrierelement 32 bildet das offenen Stirnende 29 des äußeren Kappenmantels 23. Weiters ist hier noch zu ersehen, dass der äußere Kappenmantel 23 den inneren Kappenmantel 24 in Axialrichtung überragt. Dieses Ausmaß kann der Tiefe bzw. der sich in axialer Richtung erstreckenden Länge des ersten Zentrierelements 31 entsprechen.

Zusätzlich dazu wäre es aber auch noch möglich, dass zwischen dem offenen Stirnende 29 des äußeren Kappenmantels 23 und dem Flansch 13 oder der Ringschürze 15 eine Haltevorrichtung 33 ausgebildet bzw. vorgesehen ist. Auf die Darstellung der Haltevorrichtung 33 wird der besseren Übersichtlichkeit halber verzichtet, wobei die Haltevorrichtung 33 zusammenwirkende erste und zweite Halteelemente umfassen kann. Damit kann zusätzlich zur Zentrierung der Verschlusskappe 22 bzw. der gesamten Verschlussvorrichtung 3 eine gewisse Halterung bzw. Arretierung zwischen der Verschlussvornchtung 3 und dem Probenbehälter 2, insbesondere dessen offenen Ende 4, im Übergangsbereich zwischen dem Flansch 13 und der Ringschürze 15 erfolgen.

Bevorzugt weist der Flansch 13 oder die Ringschürze 15 eine äußere Querschnittsform bzw. Außenabmessung auf, welche in etwa einer äußeren Querschnittsform einer äußeren Außenabmessung des äußeren Kappenmantels 23 entspricht. Damit können überstehende Kanten im Übergangsbereich zwischen der Ringschürze 15 bzw. dem Flanschende 14 des Flansches 13 und dem äußeren Kappenmantel 23 vermieden werden. Darüber hinaus wird so aber auch eine optisch zusammengehörige Baueinheit geschaffen. An der Ringschürze 15 kann im Bereich von dessen offenen Stirnende an deren Außenfläche ein bevorzugt über den Umfang durchlaufend ausgebildeter radial vorspringender Wulst vorgesehen sein. Dieser Wulst dient dazu, im zusammengebauten Zustand bei liegender Anordnung der Abnahmebaugruppe 1 ein direktes Aufliegen der Verschlussvorrichtung 3, insbesondere dessen Verschlusskappe 22 auf eine nicht dargestellten Auflagefläche zu vermeiden. Ist der Wulst nur bereichsweise und/oder mit einem über den Umfang gesehen zueinander unterschiedlichen radialen Überstand über die Außenfläche der Ringschürze ausgebildet, kann so eine Rollbewegung der Abnahmebaugruppe 1 verhindert werden.

Die Abmessung des inneren Kappenmantels 24 in radialer Richtung gesehen, wird bevorzugt so gewählt, dass diese in etwa der inneren Querschnittsabmessung der Innenfläche 10 der Seitenwand 7 entspricht. Bevorzugt wird die Außenabmessung jedoch geringfügig dazu kleiner gewählt. Das Septum 26 überragt bevorzugt den inneren Kappenmantel 24 in radialer Richtung. Um auch hier eine entsprechende Vorzentrierung zwischen dem Septum 26 und dem inneren Kappenmantel 24 zu erzielen, wäre es noch möglich, auch hier eine entsprechend ausgebildete Zentrieranordnung, beispielsweise in Form einer umlaufenden Ringnut, vorzusehen. Darüber hinaus wäre es aber auch möglich, nur einzelne Halteelemente am inneren Kappenmantel 24 vorzusehen, um so zusätzlich noch eine mechanische Halterung des Septums 26 an der Verschlusskappe 22, insbesondere dessen Inneren Kappenmantel 24, zu erzielen.

Wie nun besser aus den Fig. 3 und 4 zu ersehen ist, kann die Abnahmebaugruppe 1 weiters auch noch ein zusätzliches Trägerröhrchen 34 umfassen. Die Abmessungen des Trägerröhrchens 34 können jenen Standardabmessungen entsprechen, wie diese bei den üblichen Blutprobeentnahmeröhrchen angewendet werden. So kann beispielsweise der Durchmesser 13 mm oder 16 mm betragen, wobei diese eine Baulänge von beispielsweise 70 mm oder 100 mm aufweisen können.

Das Trägerröhrchen 34 umfasst einen offen ausgebildeten ersten Endbereich 35 sowie einen bevorzugt mit einem Trägerröhrchenboden 36 verschlossenen, zweiten Endbereich 37. Der zuvor beschriebene Probenbehälter 2 kann mit seiner Seitenwand 7 sowie gegebenenfalls dem daran angeordneten Ansatz 21 in den Innenraum des Trägerröhrchens 34 eingesetzt werden, wobei der offen ausgebildete, erste Endbereich 35 des Trägerröhrchens 34 in den zwischen der Außenfläche 9 der Seitenwand 7 sowie der Ringschürze 15 gebildeten, rohrförmigen Freiraum 17 hineinragt. Dabei kann der Flansch 13 als Anschlag bzw. Begrenzung für die zueinander gegenläufige Einsetzbewegung der beiden Bauteile dienen. Bei vollständigem Einsetzen kann die Stirnfläche des Trägerröhrchens 34 am Flansch 13 zur Anlage kommen bzw. gebracht werden und sich daran abstützen.

Bevorzugt weist das Trägerröhrchen 34 im Bereich seines offen ausgebildeten Endbereichs 35 sowie an seiner Außenfläche 38 eine zweite Gewindeanordnung 39 auf bzw. ist dieses damit versehen. Damit wird es für den Anwender des Probenbehälters 2 möglich, diesen zur Bildung der Abnahmebaugruppe 1, beispielsweise für die Zentrifugation des in den Sammelraum 12 bzw. Aufnahmeraum eingefüllten Blutes, diese in eine standardisierte Zentrifuge einsetzen zu können.

Aus der Fig. 4 sind die Einzelteile zur Bildung der gesamten Abnahmebaugruppe 1 in einer voneinander getrennten Stellung in Art einer in der Technik allgemein verwendeten Explosionsdarstellung gezeigt. Für die Bildung der Verschlussvorrichtung 3 ist die Verschlusskappe 22 mit dem Septum 26 zu versehen bzw. damit auszubilden. Um für die Anlieferung einen sterilen Innenraum bzw. Sammelraum 12 bereitstellen zu können, ist die Verschlussvorrichtung 3 auf das offene Ende 4 so aufzusetzen, dass einerseits die Dichtfläche 28 des Septums 26 an der Innenfläche 10 der Seitenwand 7 zur Anlage gebracht wird. Der äußere Kappenmantel 23 der Verschlusskappe 22 kann sich dabei am äußeren Übergangsbereich zwischen dem Flansch 13, insbesondere dessen Flanschende 14, und der Ringschürze 15 abstützen. Eine zusätzliche Zentrierung durch die Zentrieranordnung 30 kann dabei ebenfalls noch erfolgen.

Für die Durchführung des Aufsammelns und Einfüllen des Blutes in den Sammelraum 12 ist die Verschlussvorrichtung 3 abzunehmen und anschließend der Einfüllvorgang gegebenenfalls mit Hilfe des Aufnahmeansatzes 19 durchzuführen. Ist eine ausreichende Menge in den Sammelraum 12 eingefüllt, wird das offene Ende 4 wiederum durch die Verschlussvorrichtung 3 verschlossen.

So ist es möglich, den Probenbehälter 2 mit der darin enthaltenen Probe unmittelbar einer Zentrifugation oder anderen Auswertungsvorgängen und Analysen zu unterziehen. Dabei kann die Abstützung bzw. Anlage des Probenbehälters 2 an dem dem Boden 6 zugewendeten Stirnende bzw. der Stirnfläche der Ringschürze 15 erfolgen.

Sollte diese Abstützung bzw. Anlage der Ringschürze 15 an der hier nicht näher dargestellten Zentrifuge nicht möglich sein, kann der Probenbehälter 2 in seinem mit der Verschlussvorrichtung 3 verschlossenem Zustand in das Trägerröhrchen 34 eingesetzt werden, wie dies bereits zuvor beschrieben worden ist. Eine gegenseitige Fixierung bzw. Halterung des Probenbehälters 2 kann dabei über die zusammenwirkenden ersten und zweiten Gewindeanordnungen 18 bzw. 39 erfolgen.

Wie bereits zuvor kurz erwähnt, kann die Montage des Probenbehälters 2 am Trägerröhrchen 34 derart erfolgen, wie dies in der EP 1 711 412 B1 beschrieben worden ist. Da die Ringschürze 15 gemeinsam mit dem Flansch 13 einen kappenähnlichen Bauteil am Probenbehälter 2 ausbilden, kann grundsätzlich der Fügevorgang des Probenbehälters 2 mit dem Trägerröhrchen 34 in dazu äquivalenter Weise, jedoch an die vorhandenen Bauelemente angepasst, erfolgen.

Das Trägerröhrchen 34 entspricht dabei dem Aufnahmebehälter, wie dieser in der EP-B 1 beschrieben ist. Zum Unterschied dazu ist bei der hier vorliegenden Erfindung keine Dichtungsvorrichtung direkt innerhalb der Ringschürze 15 vorgesehen. Beim Fügevorgang wird durch die kappenähnliche Ringschürze 15 mit ihrem offenen und dem Trägerröhrchen 34 zugewendeten offenen Stirnende der offen ausgebildete erste Endbereich 35 des Trägerröhrchens 34 übergriffen.

Durch das Vorsehen der beiden Gewindeanordnungen 18, 39 mit deren zusammenwirkenden Gewindegängen zwischen der Ringschürze 15 und dem Trägerröhrchen 34 kann der Zusammenbau durch eine relative Dreh- bzw. Schwenkbewegung um die gemeinsame Längsachse 8 durchgeführt werden. Weiters können dazu auch gleichzeitig mehrere Probenbehälter 2 mit den zu fügenden Trägerröhrchen 34 zur Bildung der Abnahmebaugruppen 1 in eine gemeinsame, jedoch hier nicht näher dargestellte Montageeinrichtung verbracht werden.

Für die Fügevorgänge wird jeweils einer der zusammenzubauenden Bauteile an einem Drucklager der Montageeinrichtung drehbar um seine Längsachse 8 abgestützt, wobei durch die Montageeinrichtung auf zumindest einen der zusammenzubauenden Bauteile eine in etwa in Richtung der Längsachse 8 gerichtete Druckkraft (F) ausgeübt wird. Dabei wird durch die zusammenwirkenden Gewindegänge der Gewindeanordnungen 18, 39 die Druckkraft (F) zur Erzeugung der Relativbewegung in die relative Dreh- bzw. Schwenkbewegung um die gemeinsame Längsachse 8 umgesetzt. Während der relativen Dreh- bzw. Schwenkbewegung können die Gewindegänge der Gewindeanordnung 18, 39 über die gesamte Länge des Einschraubweges bis zum Erreichen der vollständig aufgeschraubten Position miteinander in Eingriff stehen. Diese Aufschraubbewegung kann durch Anstehen der Stirnseite des offenen ersten Endbereichs 35 des Trägerröhrchens 34 am Flansch 14 beendet sein.

Unabhängig davon wäre aber auch eine reine Steckverbindung ohne das Vorsehen der beiden Gewindeanordnungen 18 bzw. 39 möglich.

Nach erfolgtem Zentrifugiervorgang und der erfolgten Auftrennung des Blutes in dessen leichteren und schwereren Bestandteile kann beispielsweise der dem offenen Ende 4 benachbart angeordnete, leichtere Bestandteil aus dem Sammelraum 12 entnommen werden. Dies kann entweder dadurch erfolgen, dass die Verschlussvorrichtung 3 vom offenen Ende 4 des Probenbehälters 2 abgenommen wird oder dass das durchstechbare Septum 26 mit einer nicht näher dargestellten Entnahmevorrichtung aus dem Sammelraum 12 entnommen wird. Dies kann beispielsweise durch einen Absaugvorgang oder dergleichen erfolgen.

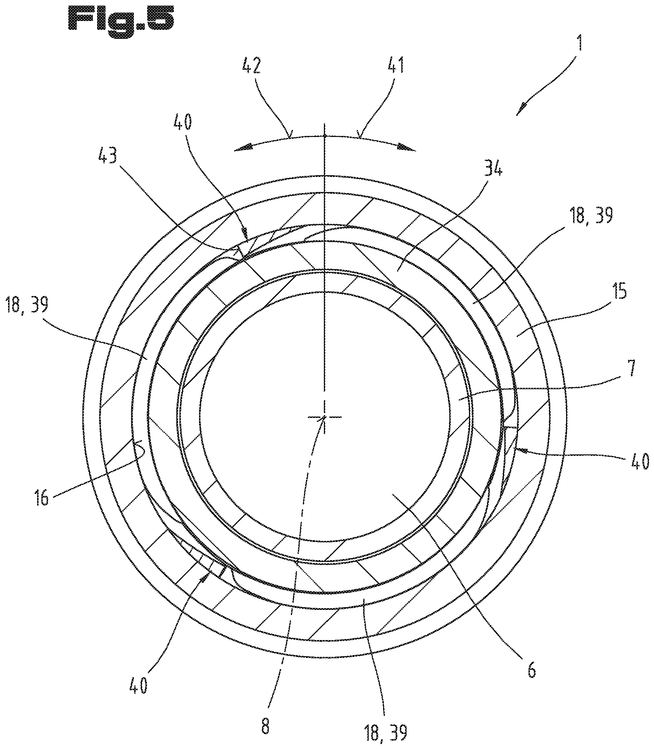

In der Fig. 5 ist eine weitere und gegebenenfalls für sich eigenständige Ausführungsform der Abnahmebaugruppe 1 mit dem in das Trägerröhrchen 34 eingesetzten , insbesondere damit über die Gewindeanordnungen 18, 39 verbundenen Probenbehälter 2 in Draufsicht geschnitten gezeigt, wobei wiederum für gleiche Teile gleiche Bezugszeichen bzw. Bauteilbezeichnungen wie in den vorangegangenen Fig. 1 bis 4 verwendet werden. Um unnötige Wiederholungen zu vermeiden, wird auf die detaillierte Beschreibung in den vorangegangenen Fig. 1 bis 4 hingewiesen bzw. Bezug genommen.

Aus manchen Gründen der nachfolgenden Probenanalyse bzw. Weiterverarbeitung sowie Weiterbehandlung des Probeninhalte der Abnahmebaugruppe 1 kann es von Nöten sein, den Probenbehälter 2 mit seiner Ringschürze 15 nach dem Einsetzen und dem Verschrauben bzw. Verbinden mit dem Trägerröhrchen 34 gegen ein unbeabsichtigtes Abschrauben bzw. Abnehmen zu sichern. Dies kann dann der Fall sein, wenn beispielsweise der Probenbehälter 2, insbesondere dessen Seitenwand 7, nicht mit einem Informationsträger versehen ist bzw. wird, sondern das Trägerröhrchen 34. Würde die Abnehmbarkeit bzw. Trennung des Probenbehälters 2 vom Trägerröhrchen 34 möglich sein, könnte der Fall auftreten, dass nur das Trägerröhrchen 34 mit der entsprechenden Information bzw. dem Informationsträger versehen ist und die im Probenbehälter 2 enthaltene Probe durch die Nichtkennzeichnung nicht mehr eindeutig einem Patienten zuordenbar ist.

Deshalb kann es vorteilhaft sein, wenn zwischen der kappenähnlichen Ringschürze 15 des Probenbehälters 2 und dem Trägerröhrchen 34 eine zusätzliche Verdrehsicherungsvorrichtung 40 vorgesehen bzw. angeordnet ist. Diese Verdrehsicherungsvorrichtung 40 kann durch unterschiedlichste zusammenwirkende Rastelemente bzw. Sicherungselemente ausgebildet sein, welche es erlauben, den Probenbehälter 2 mit dem Trägerröhrchen 34 zu fügen. Bei Vorsehen der beiden Gewindeanordnungen 18, 39 erfolgt dies durch eine Aufschraubbewegung um die Längsachse 8, wobei die Drehrichtung für den Verbindungsvorgang durch einen ersten Pfeil 41 angedeutet ist. Mit einem weiteren Pfeil 42 ist dagegen die dazu entgegengesetzt gerichtet Drehbewegung dargestellt, welche es gilt zu erschweren bzw. überhaupt zu verhindern.

Im vorliegenden Ausführungsbeispiel ist die bzw. sind die Verdrehsicherungsvornchtungen 40 an der Ringschürzenfläche 16 sowie in Richtung auf die Längsachse 8 vorragend daran ausgebildet. Um einen in Richtung des ersten Pfeils 41 ungehinderten Aufschraubvorgang zu ermöglichen, ist hier eine flache, bevorzugt stufenlose Steigung vorgesehen.

Um in der dazu entgegengesetzten Drehbewegung gemäß Pfeil 42 ein Abschrauben bzw. Abnehmen des Probenbehälters 2 vom Trägerröhrchen 34 zu verhindern, weist die Verdrehsicherungsvorrichtung 40 eine Verriegelungsfläche 43 bzw. Anschlagfläche auf. Diese ist zumeist in einer senkrecht bezüglich der Abschraubbewegung - gemäß Pfeil 42 - ausgerichteten Ebene angeordnet, um so ein Eingreifen und Anstehen z.B. auf einem der ersten Gewindegänge der Gewindeanordnungen 18 zu erzielen. Damit kann das Abschrauben erschwert oder überhaupt verhindert werden. Es könnten aber auch andere Anordnungen bzw. Ausbildungen der die Verdrehsicherungsvorrichtung 40 bildenden Elemente gewählt werden. Bei Änderung der Aufschraubrichtung, wie dies bei Rechts- oder Linksgewinden allgemein bekannt ist, ist die Anordnung sowie Ausrichtung der Verdrehsicherungsvorrichtung 40 bzw. deren Elementen an die jeweils gewählten Drehrichtungen anzupassen.

Die Ausführungsbeispiele zeigen mögliche Ausführungsvarianten der Abnahmebaugruppe 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die den eigenständigen erfinderischen Lösungen zugrundeliegende Aufgabe kann der Beschreibung entnommen werden.

Vor allem können die einzelnen in den Fig. 1, 2; 3, 4; 5 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

Der Ordnung halber sei abschließend darauf hingewiesen, dass zum besseren Verständnis des Aufbaus der Abnahmebaugruppe 1 diese bzw. deren Bestandteile teilweise unmaßstäblich und/oder vergrößert und/oder verkleinert dargestellt wurden.

**Bezugszeichenaufstellung**

| | | | |
|---|---|---|---|
| 1 | Abnahmebaugruppe | 31 | erstes Zentrierelement |
| 2 | Probenbehälter | 32 | zweites Zentrierelement |
| 3 | Verschlussvornchtung | 33 | Haltevorrichtung |
| 4 | offenes Ende | 34 | Trägerröhrchen |
| 5 | verschlossenes Ende | 35 | erster Endbereich |
| 6 | Boden | 36 | Trägerröhrchenboden |
| 7 | Seitenwand | 37 | zweiter Endbereich |
| 8 | Längsachse | 38 | Außenfläche |
| 9 | Außenfläche | 39 | zweite Gewindeanordnung |
| 10 | Innenfläche | 40 | Verdrehsicherungsvorrichtung |
| 11 | Stirnfläche | 41 | Pfeil |
| 12 | Sammelraum | 42 | Pfeil |
| 13 | Flansch | 43 | Verriegelungsfläche |
| 14 | Flanschende | | |
| 15 | Ringschürze | | |
| 16 | Ringschürzenfläche | | |
| 17 | Freiraum | | |
| 18 | erste Gewindeanordnung | | |
| 19 | Aufnahmeansatz | | |
| 20 | Aufnahmeansatzfläche | | |
| 21 | Ansatz | | |
| 22 | Verschlusskappe | | |
| 23 | äußerer Kappenmantel | | |
| 24 | innerer Kappenmantel | | |
| 25 | Stirnwand | | |
| 26 | Septum | | |
| 27 | Stirnende | | |
| 28 | Dichtfläche | | |
| 29 | Stirnende | | |
| 30 | Zentrieranordnung | | |

## Patentansprüche

1. Abnahmebaugruppe (1), insbesondere für Blutproben, mit einem Probenbehälter (2), der ein offenes Ende (4), ein durch einen Boden (6) verschlossenes Ende (5) und eine sich zwischen dem offenen Ende (4) und dem verschlossenen Ende (5) erstreckende Seitenwand (7) mit einer Außenfläche (9) und einer Innenfläche (10) aufweist, welche Seitenwand (7) im Bereich des offenen Endes (4) mit einer Stirnfläche (11) begrenzt ist, und die Seitenwand (7) sowie der Boden (6) einen Sammelraum (12) definieren, und der Probenbehälter (2) weiters an seinem mit der Stirnfläche (11) begrenzten offenen Ende (4) einen die Seitenwand (7) radial überragenden Flansch (13) sowie eine Ringschürze (15) aufweist, wobei die Ringschürze (15) an einem von der Seitenwand (7) abgewendeten Flanschende (14) des Flansches (13) angeordnet ist, und sich die Ringschürze (15) ausgehend von diesem Flanschende (14) in axialer Richtung auf das verschlossene Ende (5) erstreckt, wobei der Probenbehälter (2), der Flansch (13) und die Ringschürze (15) ein einstückiges Bauteil ausbilden, und zwischen der Außenfläche (9) der Seitenwand (7) und einer der Seitenwand (7) zugewendeten inneren Ringschürzenfläche (16) der Ringschürze (15) ein in etwa rohrförmiger Freiraum (17) ausgebildet ist, der in Axialrichtung im Bereich des offenen Endes (4) durch den Flansch (13) begrenzt ist, und mit einer Verschlussvorrichtung (3), die eine Verschlusskappe (22), sowie ein an der Verschlusskappe (22) gehaltenes, durchstechbares Septum (26) aufweist, wobei die Verschlussvorrichtung (3) in deren Verschlussstellung den Sammelraum (12) des Probenbehälters (2) im Bereich seines offenen Endes (4) abschließt, **dadurch gekennzeichnet, dass** die Verschlusskappe (22) einen äußeren Kappenmantel (23), einen in radialer Richtung davon distanziert angeordneten inneren Kappenmantel (24) sowie eine die beiden Kappenmäntel (23, 24) in radialer Richtung verbindende Stirnwand (25) umfasst und dass in der Verschlussstellung der Verschlussvorrichtung (3) ein offenes Stirnende (29) des äußeren Kappenmantels (23) im Übergangsbereich zwischen dem Flansch (13) und der Ringschürze (15) anliegt, und dass zwischen dem offenen Stirnende (29) des äußeren Kappenmantels (23) und dem Flansch (13) oder der Ringschürze (15) eine Haltevorrichtung (33) mit in der Verschlussstellung zusammenwirkenden ersten und zweiten Halteelementen vorgesehen ist und dass der Flansch (13) oder die Ringschürze (15) eine äußere Querschnittsform bzw. eine Außenabmessung aufweist, welche in etwa einer äußeren Querschnittsform bzw. einer Außenabmessung des äußeren Kappenmantels (23) entspricht und dass das Septum (26) an einem von der Stirnwand (25) distanziert angeordneten und offen ausgebildeten Stirnende (27) des inneren Kappenmantels (24) angeordnet oder ausgebildet ist.

2. Abnahmebaugruppe (1) nach Anspruch 1, **dadurch gekennzeichnet, dass** an der inneren Ringschürzenfläche (16) eine erste Gewindeanordnung (18) angeordnet oder ausgebildet ist

3. Abnahmebaugruppe (1) nach Anspruch 1 oder 2, **dadurch gekennzeichnet, dass** der Probenbehälter (2) einen insbesondere lippenförmig ausgebildeten Aufnahmeansatz (19) aufweist, welcher Aufnahmeansatz (19) mit der Seitenwand (7) verbunden ist und die Stirnfläche (11) des offenen Endes (4) in Axialrichtung auf die vom Boden (6) abgewendete Richtung überragt.

4. Abnahmebaugruppe (1) nach Anspruch 3, **dadurch gekennzeichnet, dass** sich der Aufnahmeansatz (19) nur über einen Teilabschnitt des Umfangs der Seitenwand (7) erstreckt und eine dem Sammelraum (12) zugewendete Aufnahmeansatzfläche (20) in Axialrichtung in etwa geradlinig in die durch die Seitenwand (7) gebildete Innenfläche (10) übergeht.

5. Abnahmebaugruppe (1) nach einem der vorhergehenden Ansprüche, **dadurch gekennzeichnet, dass** der Probenbehälter (2) an seinem geschlossenen Ende (5) einen rohrförmig ausgebildeten Ansatz (21) aufweist, welcher das geschlossene Ende (5) in Axialrichtung auf die vom offenen Ende (4) abgewendete Richtung überragt.

6. Abnahmebaugruppe (1) nach Anspruch 1, **dadurch gekennzeichnet, dass** das Septum (26) eine radial umlaufende Dichtfläche (28) aufweist, welche Dichtfläche (28) in der Verschlussstellung an der Innenfläche (10) der Seitenwand (7) insbesondere dichtend anliegt.

7. Abnahmebaugruppe (1) nach Anspruch 1, **dadurch gekennzeichnet, dass** zwischen dem offenen Stirnende (29) des äußeren Kappenmantels (23) und dem Flansch (13) oder der Ringschürze (15) eine Zentrieranordnung (30) mit in der Verschlussstellung zusammenwirkenden ersten und zweiten Zentrierelementen (31, 32) vorgesehen ist.

8. Abnahmebaugruppe (1) nach einem der vorhergehenden Ansprüche, **dadurch gekennzeichnet, dass** diese weiters ein Trägerröhrchen (34) umfasst, welches einen offen ausgebildeten ersten Endbereich (35) sowie einen bevorzugt mit einem Trägerröhrchenboden (36) verschlossenen zweiten Endbereich (37) aufweist, wobei der Probenbehälter (2) in das Trägerröhrchen (34) eingesetzt ist und dabei der offen ausgebildete erste Endbereich (35) des Trägerröhrchens (34) in den zwischen der Außenfläche (9) der Seitenwand (7) und der Ringschürze (15) gebildeten rohrförmigen Freiraum (17) hineinragt.

9. Abnahmebaugruppe (1) nach Anspruch 8, **dadurch gekennzeichnet, dass** das Trägerröhrchen (34) an einer Außenfläche (38) seines offen ausgebildeten ersten Endbereichs (35) mit einer zweiten Gewindeanordnung (39) versehen ist.

10. Abnahmebaugruppe (1) nach Anspruch 8 oder 9, **dadurch gekennzeichnet, dass** zwischen dem Probenbehälter (2) und dem Trägerröhrchen (34) zumindest eine Verdrehsicherungsvorrichtung (40) angeordnet oder ausgebildet ist, mittels welcher nach dem Fügen des Probenbehälters (2) mit dem Trägerröhrchen (34) eine Trennung des Probenbehälters (2) vom Trägerröhrchen (34) unterbunden ist.

11. Abnahmebaugruppe (1) nach Anspruch 10, **dadurch gekennzeichnet, dass** die zumindest eine Verdrehsicherungsvorrichtung (40) zwischen der Ringschürze (15) des Probenbehälters (2) und dem Trägerröhrchen (34) angeordnet oder ausgebildet ist.

## Claims

1. A collection assembly (1), in particular for blood samples, having a sample container (2), which has an open end (4), an end (5) closed by a base (6) and a side wall (7) extending between the open end (4) and the closed end (5) with an outer surface (9) and an inner surface (10), the side wall (7) being delimited in the region of the open end (4) by an end face (11), and the side wall (7) as well as the base (6) defining a collecting chamber (12), and the sample container (2) further comprising, at its open end (4) delimited by the end face (11), a flange (13) projecting radially over the side wall (7) as well as an annular skirt (15), wherein the annular skirt (15) is disposed on an end (14) of the flange (13) facing away from the side wall (7), and the annular skirt (15) extends from said flange end (14) in the axial direction towards the closed end (5), wherein the sample container (2), the flange (13) and the annular skirt (15) form a one-piece component, and an approximately tubular open space (17) is formed between the outer surface (9) of the side wall (7) and an inner annular skirt surface (16) of the annular skirt (15) facing the side wall (7) and is delimited by the flange (13) in the axial direction in the region of the open end (4), and having a closure device (3) which comprises a closure cap (22), as well as a penetrable septum (26) held on the closure cap (22), wherein in its closed position, the closure device (3) closes the collecting chamber (12) of the sample container (2) in the region of its open end (4), **characterized in that** the closure cap (22) comprises an outer cap casing (23), an inner cap casing (24) disposed spaced apart therefrom in the radial direction and an end wall (25) connecting the two cap casings (23, 24) in the radial direction, and **in that** in the closed position of the closure device (3), an open face end (29) of the outer cap casing (23) is seated in the intermediate region between the flange (13) and the annular skirt (15), and **in that** a holding device (33) having first and second holding elements cooperating in the closed position is provided between the open face end (29) of the outer cap casing (23) and the flange (13) or the annular skirt (15), and **in that** the flange (13) or the annular skirt (15) has an outer cross-sectional form or an external dimension which approximately corresponds to an outer cross-sectional form or an external dimension of the outer cap casing (23), and **in that** the septum (26) is disposed or formed on an open face end (27) of the inner cap casing (24) disposed spaced apart from the end wall (25).

2. The collection assembly (1) as claimed in claim 1, **characterized in that** a first threaded arrangement (18) is disposed or formed on the inner annular skirt surface (16).

3. The collection assembly (1) as claimed in claim 1 or 2, **characterized in that** the sample container (2) comprises a receiving edge (19) which in particular is in the form of a lip, the receiving edge (19) being connected to the side wall (7) and projecting the end face (11) of the open end (4) in the axial direction in the direction facing away from the base (6).

4. The collection assembly (1) as claimed in claim 3, **characterized in that** the receiving edge (19) extends over only a portion of the periphery of the side wall (7) and a receiving edge surface (20) facing the collecting chamber (12) passes in an approximately straight line in the axial direction into the inner surface (10) formed by the side wall (7).

5. The collection assembly (1) as claimed in any one of the preceding claims, **characterized in that** the closed end (5) of the sample container (2) is provided with a tubular edge (21) which projects over the closed end (5) in the axial direction in the direction facing away from the open end (4).

6. The collection assembly (1) as claimed in claim 1, **characterized in that** the septum (26) has a radially circumferential sealing surface (28), the sealing surface (28), in the closed position, bearing against the inner surface (10) of the side wall (7), in particular in a sealing manner.

7. The collection assembly (1) as claimed in claim 1, **characterized in that** a centring arrangement (30) is provided between the open face end (29) of the outer cap casing (23) and the flange (13) or the annular skirt (15) with first and second centring elements (31, 32) cooperating in the closed position.

8. The collection assembly (1) as claimed in any one of the preceding claims, **characterized in that** it further comprises a support tube (34) which has an open first end section (35) and a second end section (37) which is preferably closed by a support tube base (36), wherein the sample container (2) is inserted into the support tube (34) and the open first end section (35) of the support tube (34) projects thereby into the tubular open space (17) formed between the outer surface (9) of the side wall (7) and the annular skirt (15).

9. The collection assembly (1) as claimed in claim 8, **characterized in that** the support tube (34) is provided with a second threaded arrangement (39) on an outer surface (38) of its open first end section (35).

10. The collection assembly (1) as claimed in claim 8 or claim 9, **characterized in that** at least one anti-rotation device (40) is disposed or formed between the sample container (2) and the support tube (34) by means of which, after joining the sample container (2) to the support tube (34), a separation of the sample container (2) from the support tube (34) is prevented.

11. The collection assembly (1) as claimed in claim 10, **characterized in that** the at least one anti-rotation device (40) is disposed or formed between the annular skirt (15) of the sample container (2) and the support tube (34).

## Revendications

1. Sous-ensemble de prélèvement (1), plus particulièrement pour des échantillons de sang, avec un récipient d'échantillons (2), qui comprend une extrémité ouverte (4), une extrémité (5) fermée par un fond (6) et une paroi latérale (7), s'étendant entre l'extrémité ouverte (4) et l'extrémité fermée (5), avec une surface externe (9) et une surface interne (10), cette paroi latérale (7) étant limitée, au niveau de l'extrémité ouverte (4), par une surface frontale (11) et la paroi latérale (7) ainsi que le fond (6) définissant un espace de collecte (12) et le récipient d'échantillons (2) comprend en outre, au niveau de son extrémité ouverte (4), limitée par la surface frontale (11), une bride (13) dépassant radialement de la paroi latérale (7) ainsi qu'un tablier annulaire (15), le tablier annulaire (15) étant disposé au niveau d'une extrémité (14) de la bride (13), opposée à la paroi latérale (7) et le tablier annulaire (15) s'étendant, à partir de cette extrémité de bride (14), dans la direction axiale vers l'extrémité fermée (5), le récipient d'échantillons (2), la bride (13) et le tablier annulaire (15) formant un composant d'une seule pièce et, entre la surface externe (9) de la paroi latérale (7) et une surface interne (16) du tablier annulaire (15), orientée vers la paroi latérale (7), un espace libre tubulaire (17) étant formé, qui est limité, dans la direction axiale au niveau de l'extrémité ouverte (4), par la bride (13) et avec un dispositif de fermeture (3), qui comprend un capuchon de fermeture (22) ainsi qu'une cloison perforable (26), maintenue sur le capuchon de fermeture (22), le dispositif de fermeture (3) fermant, dans sa position de fermeture, l'espace de collecte (12) du récipient d'échantillons (2) au niveau de son extrémité ouverte (4), **caractérisé en ce que** le capuchon de fermeture (22) comprend une enveloppe externe de capuchon (23), une enveloppe interne de capuchon (24), éloignée de celle-ci dans la direction axiale, ainsi qu'une paroi frontale (25) reliant les deux enveloppes de capuchon (23, 24) dans la direction radiale et **en ce que**, dans la position de fermeture du dispositif de fermeture (3), une extrémité frontale (29) de l'enveloppe externe de capuchon (23) s'appuie dans la zone de transition entre la bride (13) et le tablier annulaire (15) et **en ce que**, entre l'extrémité frontale ouverte (29) de l'enveloppe externe de capuchon (23) et la bride (13) ou le tablier annulaire (15), est disposée un dispositif de maintien (33) avec un premier et un deuxième éléments de maintien interagissant dans la position de fermeture et **en ce que** la bride (13) ou le tablier annulaire (15) présente une forme de section externe ou une dimension externe qui correspond approximativement à une forme de section externe ou à une dimension externe de l'enveloppe externe de capuchon (23) et **en ce que** la cloison (26) est disposée ou formée au niveau d'une extrémité frontale (27), disposée de manière distante par rapport à la paroi frontale (25) et conçu de manière ouverte, de l'enveloppe interne de capuchon (24).

2. Sous-ensemble de prélèvement (1) selon la revendication 1, **caractérisé en ce que**, au niveau de la surface interne du tablier annulaire (16) est disposé ou formé un dispositif fileté (18).

3. Sous-ensemble de prélèvement (1) selon la revendication 1 ou 2, **caractérisé en ce que** le récipient d'échantillons (2) comprend un embout de prélèvement (19) conçu en forme de lèvres, cet embout de prélèvement (19) étant relié avec la paroi latérale (7) et la surface frontale (11) de l'extrémité ouverte (4) dépassant, dans la direction axiale, dans la direction opposée au fond (6).

4. Sous-ensemble de prélèvement (1) selon la revendication 3, **caractérisé en ce que** l'embout de prélèvement (19) s'étend uniquement sur une portion de la circonférence de la paroi latérale (7) et une surface d'embout de prélèvement (20), orientée vers l'espace de collecte (12), se transformant, dans la direction axiale, approximativement en ligne droite, dans la surface interne (10) formée par la paroi latérale (7).

5. Sous-ensemble de prélèvement (1) selon l'une des revendications précédentes, **caractérisé en ce que** le récipient d'échantillons (2) comprend, au niveau de son extrémité fermée (5), un embout tubulaire (21), qui dépasse de l'extrémité fermée (5) dans la direction axiale, dans la direction opposée à l'extrémité ouverte (4).

6. Sous-ensemble de prélèvement (1) selon la revendication 1, **caractérisé en ce que** la cloison (26) comprend une surface d'étanchéité radiale circulaire (28), cette surface d'étanchéité (28) s'appuyant plus particulièrement de manière étanche, dans la position de fermeture, contre la surface interne (10) de la paroi latérale (7).

7. Sous-ensemble de prélèvement (1) selon la revendication 1, **caractérisé en ce que**, entre l'extrémité frontale ouverte (29) de l'enveloppe externe de capuchon (23) et la bride (13) ou le tablier annulaire (15) se trouve un dispositif de centrage (30) avec des premiers et des deuxièmes éléments de centrage (31, 32) interagissant dans la position de fermeture.

8. Sous-ensemble de prélèvement (1) selon l'une des revendications précédentes, **caractérisé en ce que** celui-ci comprend en outre un tube de support (34) qui comprend une première zone d'extrémité (35), conçue de manière ouverte, ainsi qu'une deuxième zone d'extrémité (37) fermée de préférence avec un fond de tube de support (36), le récipient d'échantillons (2) étant inséré dans le tube de support (34) et la première zone d'extrémité (35), conçue de manière ouverte, du tube de support (34), dépassant dans l'espace libre tubulaire (17) formé entre la surface externe (9) de la paroi latérale (7) et le tablier annulaire (15).

9. Sous-ensemble de prélèvement (1) selon la revendication 8, **caractérisé en ce que** le tube de support (34) est muni, au niveau d'une surface externe (38) de sa première zone d'extrémité (35), conçue de manière ouverte, d'un dispositif fileté (39).

10. Sous-ensemble de prélèvement (1) selon la revendication 8 ou 9, **caractérisé en ce que**, entre le récipient d'échantillons (2) et le tube de support (34), est disposé ou formé au moins un dispositif anti-rotation (40), qui permet d'éviter, après l'assemblage du récipient d'échantillons (2) avec le tube de support (34), une séparation du récipient d'échantillons (2) du tube de support (34).

11. Sous-ensemble de prélèvement (1) selon la revendication 10, **caractérisé en ce que** l'au moins un dispositif anti-rotation (40) est disposé ou formé entre le tablier annulaire (15) du récipient d'échantillons (2) et le tube de support (34).
